# EUROPEAN PATENT SPECIFICATION

(11) **EP 3 492 014 B1**
(45) Date of publication and mention of the grant of the patent: **19.10.2022**
(21) Application number: 18207668.7
(22) Date of filing: 21.11.2018
(51) Int. Cl.: A61B 5/1459, A61B 5/1473

(54) **MULTIMODAL ANALYTE SENSORS FOR MEDICAL DEVICES**
MULTIMODALE ANALYTSENSOREN FÜR MEDIZINISCHE VORRICHTUNGEN
CAPTEURS D 'ANALYTES MULTIMODAUX POUR DISPOSITIFS MÉDICAUX

(30) Priority: 01.12.2017 CN 201711249985; 20.09.2018 US 201816136875
(43) Date of publication of application: 05.06.2019
(73) Proprietor: Cardiac Pacemakers, Inc., St. Paul, MN 55112-5798 (US)
(72) Inventor: Li, Yingbo, Changning District, Shanghai 201103 (CN); Kane, Michael J., St. Paul, MN Minnesota 55105 (US); Maile, Keith R., New Brighton, MN Minnesota 55112 (US)
(74) Representative: Pfenning, Meinig & Partner mbB

(56) References cited:
- WO-A1-2007/137037
- US-A1- 2009 076 353
- US-A1- 2014 357 964
- US-A1- 2016 374 597

## Description

### Field

Embodiments herein relate to multimode analyte sensors. In particular, embodiments herein relate to multimode analyte sensors for use with implantable medical devices.

### Background

In the context of diagnosis and monitoring of patients, clinicians frequently evaluate many different pieces of data about their patients including physical observations, descriptions of symptoms, test results, and the like. One aspect that testing can reveal is the physiological concentration of chemical analytes such as electrolytes for the patient. Chemical analyte concentrations can be important to know because of their effect on various organs and bodily functions. In many cases, chemical analyte concentrations are assessed by drawing a fluid sample (or other sample) from the patient followed by an *in vitro* assay. For instance, document WO 2007/137037 A1 relates to an implantable medical device with a chemical sensor.

### Summary

Embodiments herein relate to implantable multimode analyte sensors. The invention is defined by claim 1. Preferred embodiments are defined in the dependent claims. Further aspects, examples and embodiments disclosed herein are for exemplary purpose only and do not form part of the invention.

### Brief Description of the Figures

FIG. 1 is a schematic top view of an implantable medical device .
FIG. 2 is a schematic view of analyte sensors.
FIG. 3 is a cross-sectional view of analyte sensors taken along line 3-3' of FIG. 2.
FIG. 4 is a schematic cross-sectional view of an analyte sensor
FIG. 5 is a schematic cross-sectional view of an analyte sensor .
FIG. 6 is a schematic cross-sectional view of an analyte sensor .
FIG. 7 is a schematic cross-sectional view of an analyte sensor.
FIG. 8 is a schematic cross-sectional view of an analyte sensor.
FIG. 9 is a schematic cross-sectional view of an implantable medical device .
FIG. 10 is a schematic diagram of components of an implantable medical device.

### Detailed Description

Implantable analyte sensors can be used to gather data about physiological analytes while a patient is away from a medical care facility and without needing to draw blood or another fluid from the patient. The clinical value of data provided by implanted sensors increases (possibly exponentially) with the number of different analytes that can be observed simultaneously.

However, maximum device volume, complexity and surface area are severely constrained in the context of implanted devices. As such, the inclusion of additional sensors to increase the number of different analytes detected can negatively affect physical dimensions, circuit complexity, manufacturability, durability, yield and usability.

In accordance with embodiments herein, multimodal sensors (and in particular chronically implanted multimodal sensors) can be used to detect multiple different analytes with each sensing element and thus allow for the detection of multiple different analytes without substantially increasing the overall complexity, size and power consumption of the device.

In various embodiments herein, implantable medical devices are equipped with multimodal analyte sensors. In particular, various analyte sensors herein can operate using both colorimetric sensing and photoluminescent sensing. Colorimetric sensing is accomplished using an indicator or moiety that changes its color upon binding with an analyte of interest and the resulting color change is then captured. Photoluminescent sensing (including, but not limited to, fluorescence and phosphorescence) operates very differently than colorimetry. Photoluminescent sensing is accomplished using an indicator or moiety that involves optical absorption and re-emission (typically at a longer wavelength) via a phosphor. Multimodal chemical (or analyte) sensors herein, such as bimodal sensors, can include both colorimetric sensing components and photoluminescent sensing components within the same sensing element such that the number of analytes can be multiplied without increasing the overall size and complexity of the device to the same degree that would otherwise be required if only unimodal analyte sensors were used.

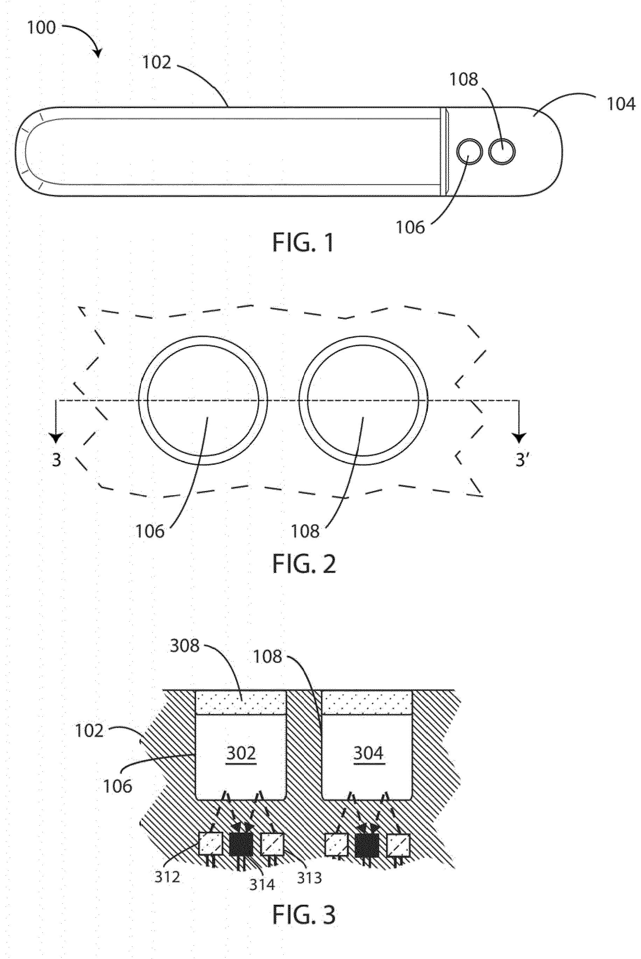

Referring now to FIG. 1, an implantable medical device (IMD) 100 is shown in accordance with the embodiments herein. The IMD 100 can include an implantable housing 102 and a header 104 coupled to the implantable housing 102. Various materials can be used. However, in some embodiments, the housing 102 can be formed of a material such as a metal, ceramic, a polymer, or a composite. The header 104 can be formed of various materials, but in some embodiments the header 104 can be formed of a polymer (translucent or opaque) such as an epoxy material. In some cases, optical paths within the header 104 can be provided by internal subcomponents employing either transparent or reflective coupling modes. In some embodiments, the header 104 can be hollow. In other embodiments, the header 104 can be filled with components and/or structural materials such as epoxy or another material such that it is non-hollow.

The IMD 100 can also include analyte sensors 106 and 108 coupled to the implantable housing 102. Analyte sensors 106, 108 can each be configured to detect one or more analytes, such as an ion concentration of a bodily fluid, when implanted in the body. In various embodiments herein, analyte sensors 106, 108 are multimodal sensors as described more fully below. Bodily fluids can include blood, interstitial fluid, serum, lymph, serous fluid, cerebrospinal fluid, and the like.

In some embodiments, analyte sensors 106, 108 can each be configured to detect two or more analytes selected from ions, electrolytes, proteins, sugars, hormones, peptides, amino acids, metabolic products or the like. In some embodiments, the analyte sensors 106, 108 can be configured to detect an ion selected from the group consisting of potassium, sodium, calcium, magnesium, lithium, hydronium, hydrogen phosphate, chloride, bicarbonate, and the like. In some embodiments, the sensors 106, 108 can be configured to detect creatinine or glucose. However, many other physiological analytes are also contemplated herein and are discussed further below.

It will be appreciated that the analyte sensors 106, 108 can be positioned at any location along IMD 100, including along the implantable housing 102 and along the header 104. It will also be appreciated that though FIG. 1 shows a device having two multimodal analyte sensors 106, 108, any number of analyte sensors can be present. For example, the device can include at least 1, 2, 3, 4, 5, 6, 7, 8, 9, 10, 11, 12, or more analyte sensors, or a number of sensors falling within a range between any of the foregoing. In some embodiments the analyte sensors 106, 108 can be configured to detect the same analyte or same two analytes, whereas in other embodiments, the analyte sensors 106, 108 can be configured to detect different analytes.

The IMD 100 can take on various dimensions. In a particular embodiment herein, the IMD 100 can be approximately 2 to 3 inches in length, 0.4 to 0.6 inches wide, and 0.15 to 0.35 inches thick. However, in some embodiments, the IMD 100 can be about 0.25, 0.5, 1.0, 2.0, 3.0, 4.0, or 5.0 inches in length. In some embodiments, the length can be in a range wherein any of the foregoing lengths can serve as the upper or lower bound of the range, provided that the upper bound is greater than the lower bound. In some embodiments, the IMD 100 can be about 0.25, 0.5, 0.75, 1.0, or 2.0 inches in width. In some embodiments, the length can be in a range wherein any of the foregoing widths can serve as the upper or lower bound of the range, provided that the upper bound is greater than the lower bound. In some embodiments, the IMD 100 can be about 0.10, 0.25, 0.50, 0.75 or 1.0 inches thick (1 in = 2,54 cm)

In some embodiments, the thickness can be in a range wherein any of the foregoing thicknesses can serve as the upper or lower bound of the range, provided that the upper bound is greater than the lower bound.

Referring now to FIG. 2, a top-down view of the analyte sensors 106, 108 is shown magnified with respect to FIG. 1. Analyte sensors 106, 108 are shown as circles, however, it will be appreciated that the analyte sensors embodied herein can take on many geometric shapes and sizes, including but not limited to squares, ovals, triangles, rectangles, pentagons, octagons, parallelograms, etc.

Referring now to FIG. 3, a cross-sectional view of analyte sensors 106, 108 along line 3-3' of FIG. 2 is shown. Analyte sensors 106 and 108 can include, but are not limited to, sensing elements 302 and 304, respectively. Each of the analyte sensors 106, 108 can also include an analyte diffusion window 308 (or simply analyte window) disposed at the top of sensing elements 302, 304.

Each analyte window 308 can be formed from a permeable material, such as an ion permeable polymeric matrix material. Many different materials can be used as the ion permeable polymeric matrix material. In some embodiments, the ion permeable polymeric matrix material can be a hydrogel. In some embodiments, the ion permeable polymeric matrix material can be polyhydroxyethyl methacrylate (polyHEMA) either as a homopolymer or a copolymer including the same. The ion permeable polymeric matrix material(s) can be chosen based on its permeability to one or more of an electrolyte, a protein, a sugar, a hormone, a peptide, an amino acid, or a metabolic product. Specific ion permeable polymeric matrix material are discussed in more detail below. In some embodiments, the analyte window 308 can be opaque to the passage of light in one or more of the visible, ultraviolet (UV), or near-infrared (NIR) frequency spectrums.

FIG. 3 shows analyte sensors 106, 108 as optical analyte sensors. The optical analyte sensors can include optical excitation assemblies 312, 313 and optical detection 314 assemblies. Suitable optical excitation and optical detection assemblies for use herein are discussed in more detail below.

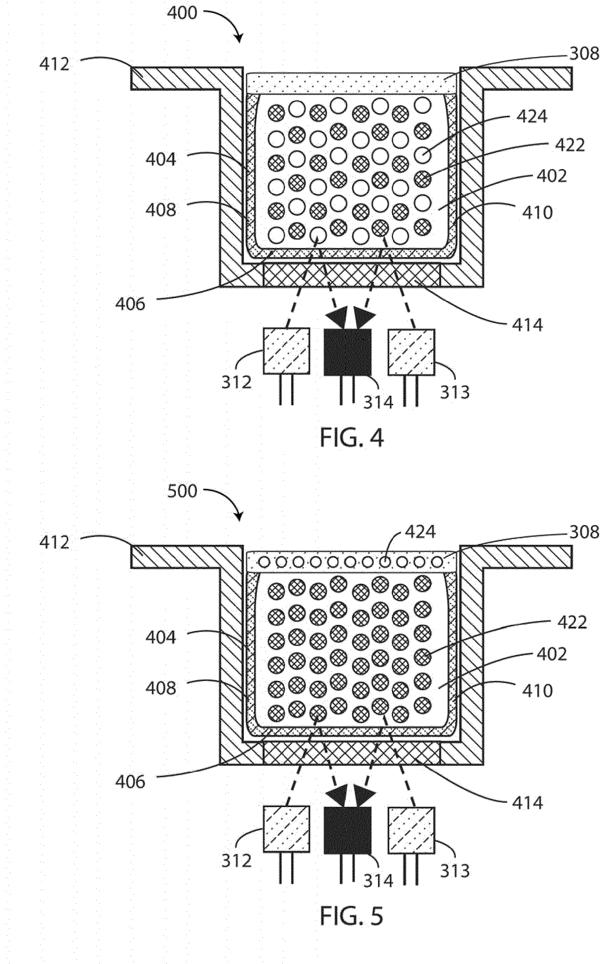

While FIG. 3 shows light rays reflecting and/or absorbing near the bottom of the sensing elements, it will be appreciated that this is merely for ease of illustration and that the entire sensing element can be exposed to light from the optical excitation assemblies and therefore contribute to generating a response that can be detected by the optical detection assemblies (with similar comments applying to FIGS. 4, 5, 6, 7 and 9).

In accordance with the embodiments herein, some of the analyte sensors can also include a bioerodible masking layer disposed on or over the exterior surface of the analyte sensor or the analyte window (if one is included) so as to shield the analyte sensors from the implant environment until a predetermined point in time. Masking layer can be formed from various bioerodible polymers including, but not limited to, polylactic acid, poly-L-lactic acid, and derivatives thereof. Additional masking layer materials and their physical properties suitable for use with the embodiments herein are described more fully below.

Referring now to FIG. 4, a cross-sectional view of an analyte sensor 400 in accordance with the embodiments herein is shown. Analyte sensor 400 can include sensing element 402 and analyte window 308, which can be part of the sensing element 402. The sensing element 402 can include an outer barrier layer 404 formed, in full or in part, from a permeable material, such as the ion permeable polymeric matrix materials as described below. In some embodiments, at least a portion of the outer barrier layer 404 is formed of polyHEMA, agarose, alginates, polyvinyl alcohol, polyacrylate, collagen, PEG, gelatin, glass, borosilicate glass, or mixtures or derivatives thereof. Outer barrier layer 404 can include a bottom 406, and opposed sides 408 and 410 to surround an interior volume of sensing element 402. In some embodiments, analyte window 308 is integrated with and/or form the top of the outer barrier layer 404 and in some cases is the same material as the other portions of the outer barrier layer 404. In other embodiments, analyte window 308 can be formed from a different material than the outer barrier layer 404. In some embodiments, only the analyte window 308 is permeable to an analyte. In some alternative embodiments, the entire outer barrier layer 404 is permeable to an analyte. In some embodiments, the analyte window can be created from the ion permeable polymeric matrix material, polyhydroxyethyl methacrylate (polyHEMA).

In some embodiments, the implantable housing 102 (see FIG. 1) can include a recessed pan 412 into which the sensing element 402 fits. In some embodiments, the top of the recessed pan 412 can be substantially flush with the top of the sensing element 402. In other embodiments, the top of the recessed pan 412 can be higher than the top of the sensing element 402 so as to create a space between the top of the sensing element 402 and the top of the recessed pan.

In some embodiments, the sensing element 402 may lack an outer barrier layer and the surfaces of the recessed pan 412 can be used to hold the sensor element components (e.g., recessed pan 412 itself can serve as an outer barrier layer).

In some embodiments, implantable housing 102 (see FIG. 1) can define an aperture occluded by a transparent member 414. The transparent member 414 can be a glass (including but not limited to borosilicate glasses), a polymer or other transparent material. The aperture can be disposed at the bottom of the recessed pan 412. The aperture can provide an interface allowing for optical communication between sensing element 402 and the optical excitation 312, 313 and optical detection 314 assemblies.

It will be appreciated that outer barrier layer 404, or portions thereof such as the bottom 406, can be made from a transparent polymer matrix material to allow for optical communication between the sensing element 402 and optical excitation 312, 313 and optical detection 314 assemblies. In will be appreciated, however, that bottom 406 of sensing element 402 may or may not be a discrete material or layer. For example, in some embodiments, bottom 406 and the transparent member 414 may be layers of different materials or may be the same type of material.

The optical excitation assemblies 312, 313 can be configured to illuminate the sensing element 402. Optical excitation assemblies 312, 313 can include a light source (or electromagnetic wave emitter) such as a light emitting diode (LED), vertical-cavity surface-emitting lasers (VCSELs), electroluminescent (EL) devices, and the like. Optical excitation assemblies herein can include broadband emitters and narrowband emitters. In some embodiments, optical excitation assemblies herein can emit electromagnetic waves primarily at a particular frequency or frequency band (primary frequency). Some optical excitation assemblies herein can produce light within the visible spectrum (e.g., 395 nm to 700 nm) and some can produce light within the ultraviolet (UV) spectrum (e.g., 10 nm to 395 nm) or near-infrared (NIR) spectrum (e.g., 700 nm to 1.5 or 2.0 µm). Some optical excitation assemblies herein can produce electromagnetic waves at frequencies outside of the preceding frequency ranges. In some embodiments, each optical excitation assembly can include a single emitter. In other embodiments, each optical excitation assembly can include more than one emitter, such as 2, 3, 4, 5, 6, 7, 8 or more emitters. In some particular embodiments, each analyte (or chemical) sensor herein can include one or two emitters operating within the visible spectrum and at least one emitter selected from those operating with the UV spectrum and those operating within the near-infrared (NIR) spectrum.

The optical detection assembly 314 can receive light and generate a signal. In some embodiments, an optical filter, such as a low pass filter or a band pass filter can be disposed over at least one of the optical detection assemblies. The optical detection assembly 314 can be in communication with other circuitry of the device in order to deliver the signal for further processing. Optical detection assembly 314 can include a component selected from the group consisting of a photodiode, a phototransistor, a charge-coupled device (CCD), a junction field effect transistor (JFET) optical sensor, a complementary metal-oxide semiconductor (CMOS) optical sensor, an integrated photo detector integrated circuit, a light to voltage converter, and the like. Optical excitation 312, 313 and optical detection 314 assemblies are discussed in further detail below. Briefly, however, the optical excitation assemblies can be configured to emit light at one or more visible spectrum frequencies, at one or more ultraviolet frequencies, or one or more near-infrared (NIR) frequencies. While two optical excitation assemblies are shown in combination with a single optical detection assembly, it will be appreciated that the numbers of excitation assemblies and detection assemblies are not so limited. Indeed, various numbers of excitation assemblies and detection assemblies are specifically contemplated herein.

The sensing element 402 can be multimodal. By way of example, the sensing element 402 can be both colorimetric and photoluminescent. For example, the sensing element 402 can include a colorimetric response element specific for a first chemical analyte and a photoluminescent response element specific for a second chemical analyte. In some cases, the photoluminescent response element is fluorescent. In some cases, the photoluminescent response element is phosphorescent. As such, the sensing element can include response elements of a first type 422 and response elements of a second type 424. In some cases, response elements of the first type 422 can be colorimetric while the response elements of the second type 424 can be photoluminescent. In some cases, response elements of the first type 422 can be photoluminescent while the response elements of the second type 424 can be colorimetric. In some embodiments, both the response elements of the first type 422 and the response elements of the second type 424 can be fluorescent, but they can operate in a non-overlapping manner, such that one is triggered by wavelength A and one is triggered by wavelength B, and wavelengths A and B are not the same.

In the example illustrated by FIG. 4, the response elements (such as the colorimetric response elements and the photoluminescent response elements) are both disposed throughout (such as evenly dispersed throughout) the interior volume of the sensing element 402. However, in some embodiments, the response elements may be only disposed in certain portions of the interior volume of the sensing element 402. In some embodiments, response elements of the first type 422 and response elements of the second type 424 can be segregated from one another into different portions of the interior volume of the sensing element 402.

The response elements (first type 422 and second type 424) in FIG. 4 are shown in the form of beads. The beads can include a polymer matrix (e.g., polymeric beads), porous glass material, or another type of porous or non-porous material. Various chemistries described below can be disposed within and/or bonded to the polymer matrix of porous glass material. The beads can have various diameters which can be all the same or can be different, such as different between different response element types). However, it will be appreciated that beads are merely one physical form that the response elements can take. Indeed, many different shapes and forms of response elements are contemplated herein.

In some embodiments, all of the response elements can be disposed within the interior volume of the multimode sensing element. However, in other embodiments, at least some of the response elements can be disposed within another portion such as the outer barrier layer or the analyte window.

Referring now to FIG. 5, a schematic cross-sectional view of an analyte sensor 500 in accordance with various embodiments herein. The analyte sensor 500 can include sensing element 402 and analyte window 308, which can be part of the sensing element 402. The sensing element 402 can include an outer barrier layer 404 formed, in full or in part, from a permeable material, such as the ion permeable polymeric matrix materials as described below. Outer barrier layer 404 can include a bottom 406, and opposed sides 408 and 410 to surround an interior volume of sensing element 402. In some embodiments, analyte window 308 can be integrated with and/or form the top of the outer barrier layer 404 and, in some cases, is the same material as the other portions of the outer barrier layer 404. In other embodiments, analyte window 308 can be formed from a different material than the outer barrier layer 404.

In some embodiments, the implantable housing 102 (shown in FIGS. 1 and 3) can include a recessed pan 412 into which the sensing element 402 fits. In some embodiments, implantable housing 102 can define an aperture occluded by a transparent member 414. The optical excitation assemblies 312, 313 can be configured to illuminate the sensing element 402. Optical detection assembly 314 can be configured to detect light reflected from or emitted by the sensing element 402.

The sensing element 402 can be multimodal. The sensing element 402 can include response elements of a first type 422 and response elements of a second type 424. Response elements of the first type 422 can be colorimetric while the response elements of the second type 424 can be photoluminescent. In some cases, response elements of the first type 422 can be photoluminescent while the response elements of the second type 424 can be colorimetric.

In the example illustrated by FIG. 5, the response elements of the first type 422 are disposed within the interior volume of the sensing element 402 while the response elements of the second type 424 are disposed within the analyte window 308. Although the response elements of the second type 424 within the analyte window 308 are shown as beads, it will be appreciated that the response element may simply be integrated throughout and/or bonded to the material forming the analyte window 308. In some alternative embodiments, the response elements of the second type 424 are disposed elsewhere within the outer barrier layer 404.

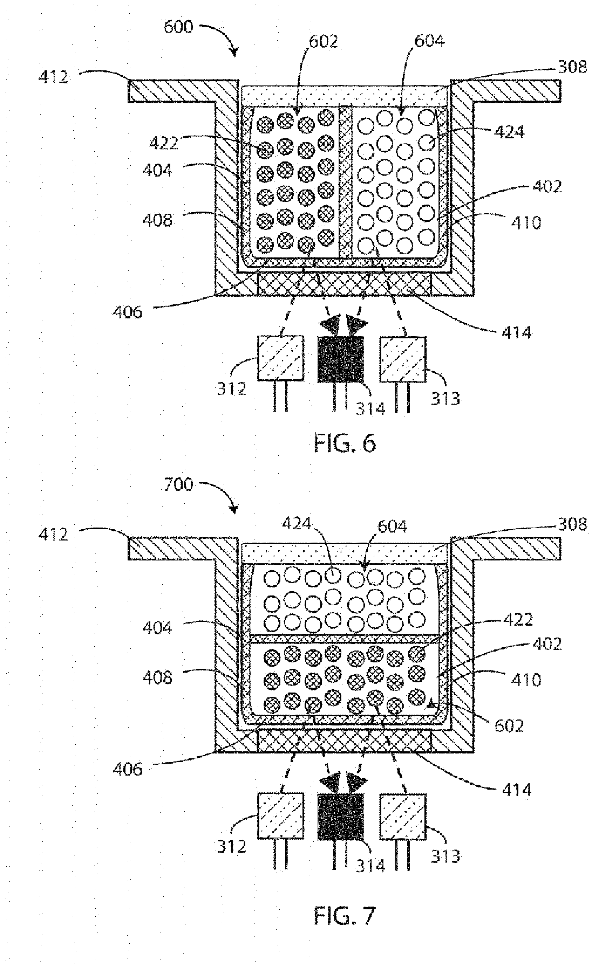

In some embodiments, the interior volume of the sensing element can be divided into two or more chambers. Referring now to FIG. 6, a schematic cross-sectional view of an analyte sensor 600 in accordance with various embodiments herein. The analyte sensor 600 can include sensing element 402 and analyte window 308, which can be part of the sensing element 402. The sensing element 402 can include an outer barrier layer 404 formed, in full or in part, from a permeable material, such as the ion permeable polymeric matrix materials as described below. Outer barrier layer 404 can include a bottom 406, and opposed sides 408 and 410 to surround an interior volume of sensing element 402.

The interior volume of sensing element 402 can include a first chamber 602 and a second chamber 604 separate from the first chamber 602. The sensing element 402 can include response elements of a first type 422 and response elements of a second type 424. Response elements of the first type 422 can be colorimetric while the response elements of the second type 424 can be photoluminescent. In some cases, response elements of the first type 422 can be photoluminescent while the response elements of the second type 424 can be colorimetric. The response elements of the first type 422 can be disposed within the first chamber 602. The response elements of the second type 424 can be disposed within the second chamber 604. FIG. 7 shows a schematic cross-sectional view of an analyte sensor 700 in accordance with various embodiments herein. In FIG. 7, the first chamber 602 and the second chamber 604 are arranged on top of one another.

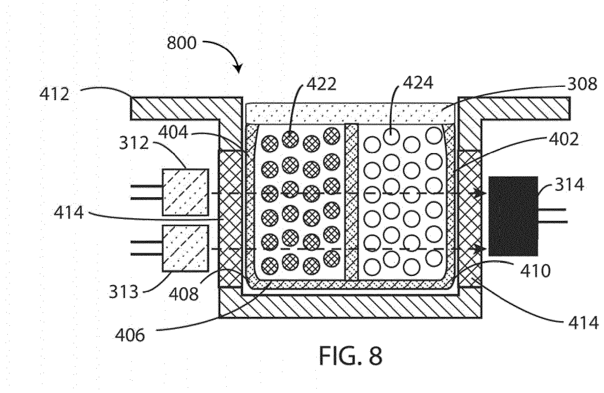

FIG. 8 shows similar components to that shown in FIG. 6. However, in the alternative analyte sensor 800 of FIG. 8, the optical excitation 312, 313 and optical detection 314 assemblies are located on opposite lateral sides of the sensing element 402. In addition, two transparent members 414 are included, disposed on or within opposed lateral sides of the recessed pan 412. Many different configurations are contemplated herein.

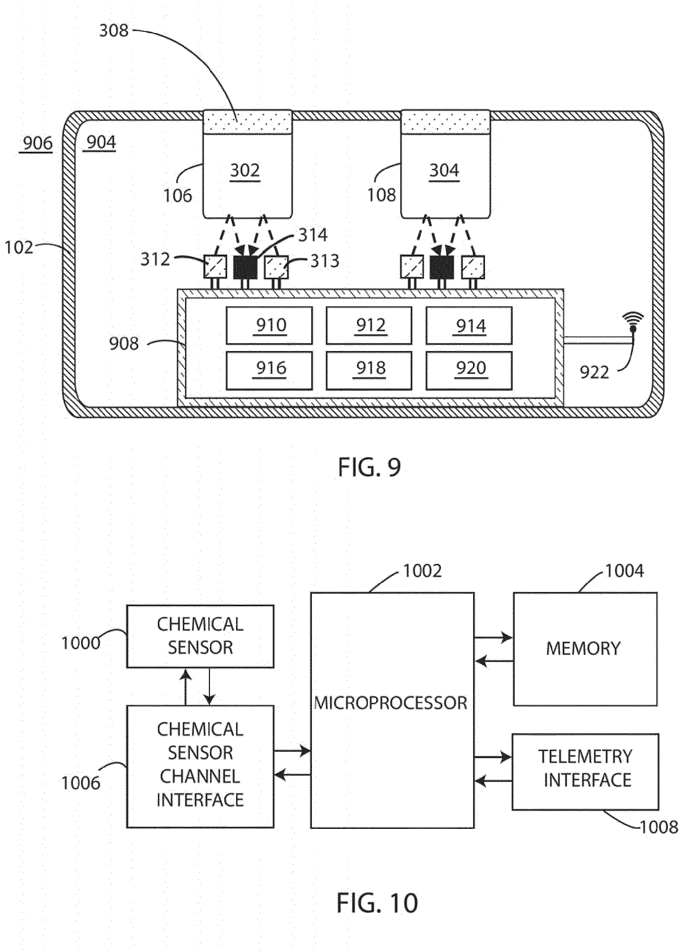

Referring now to FIG. 9, a schematic cross-sectional view of an IMD in accordance with various embodiments herein is shown. The IMD can include implantable housing 102. The implantable housing 102 of can include various materials such as metals, polymers, ceramics, and the like. In some embodiments, the implantable housing 102 can be a single integrated unit. In other embodiments, the implantable housing 102 can include implantable housing 102 and header 104 (not shown), as discussed above. In some embodiments, the implantable housing 102, or one or more portions thereof, can be formed of titanium. In some embodiments, one or more segments of the implantable housing 102 can be hermetically sealed.

Implantable housing 102 can define an interior volume 904 that in some embodiments is hermetically sealed off from the area 906 outside of the IMD. The IMD can include circuitry 908, which can be disposed within the interior volume 904, within the header (see 104 in FIG. 1), or distributed between both. Circuitry 908 can include various components, such as components 910, 912, 914, 916, 918, and 920. In some embodiments, these components can be integrated and in other embodiments these components can be separate. In some embodiments, the components can include one or more of a microprocessor, memory circuitry (such as random access memory (RAM) and/or read only memory (ROM)), recorder circuitry, telemetry circuitry, analyte sensor interface circuitry, power supply circuitry (which can include one or more batteries), normalization circuitry, analyte sensor control circuitry, and the like. In some embodiments recorder circuitry can record the data produced by the analyte sensor and record time stamps regarding the same. In some embodiments, the circuitry can be hardwired to execute various functions, while in other embodiments the circuitry can be implemented as instructions executing on a microprocessor or other computation device.

The circuitry 908 can include a control circuit and can be configured to receive (directly or indirectly) a signal from the optical detection assembly or cause another component to receive a signal from the optical detection assembly.

In some embodiments, the circuitry 908 can be configured to calculate a concentration of two or more analytes using signals received from the optical detection assembly. In the context of a colorimetric mode, this can be done by comparing the amplitude of signals at two or more frequencies and then calculating a concentration of the analyte based on the comparison. Many different specific techniques can be used for calculating a concentration based on a colorimetric signal or signals. In the context of a photoluminescent mode, this can be done by measuring the amplitude of a signal(s) at a characteristic response frequency wherein the amplitude correlates to a concentration of a particular analyte. However, in other cases, concentration of the analyte can be determined by calculating a response-time constant and then determining a concentration based on a correlation to the calculated response-time constant. Many different specific techniques can be used for calculating a concentration based on a photoluminescent signal or signals.

It will be appreciated that signal(s) herein can be subject to various intermediate steps including, but not limited to, filtering, amplification, analog to digital conversion, digital to analog conversion, and the like.

A telemetry interface 922 can be provided for communicating with external devices such as a programmer, a home-based unit, and/or a mobile unit (e.g., a cellular phone, portable computer, etc.). In some embodiments telemetry interface 922 can be provided for communicating with implanted devices such as a therapy delivery device (e.g. a pacemaker or cardioverter-defibrillator) or a monitoring-only device (e.g. an implantable loop recorder). In some embodiments, the circuitry can be implemented remotely, via either near-field, far-field, conducted, intra-body or extracorporeal communication, from instructions executing on any of the external or the implanted devices, etc. In some embodiments, the telemetry interface 922 can be located within implantable housing 102. In some embodiments, the telemetry interface 922 can be located in header 104.

The optical excitation 312, 313 and optical detection 314 assemblies of the analyte sensors embodied herein can be in electrical communication with the circuitry 908 within the interior volume 904. In some embodiments, the control circuitry 908 can be configured to selectively activate the optical excitation 312, 313 and optical detection 314 assemblies of the analyte sensors embodied herein.

Referring now to FIG. 10, a schematic diagram of components of the implantable medical devices in accordance with various embodiments herein. It will be appreciated that some embodiments can include additional elements beyond those shown in FIG. 10. In addition, some embodiments may lack some elements shown in FIG. 10. The IMD can gather information through one or more sensing channels. A microprocessor 1002 can communicate with a memory 1004 via a bidirectional data bus. The memory 1004 can include read only memory (ROM) or random access memory (RAM) for program storage and RAM for data storage, or any combination thereof. The implantable medical device can also include one or more analyte sensors 1000 and one or more analyte sensor channel interfaces 1006 which can communicate with a port of microprocessor 1002. The analyte sensor channel interface 1006 can include various components such as analog-to-digital converters for digitizing signal inputs, sensing amplifiers, registers which can be written to by the control circuitry in order to adjust the gain and threshold values for the sensing amplifiers, source drivers, modulators, demodulators, multiplexers, and the like. A telemetry interface 1008 is also provided for communicating with external devices such as a programmer, a home-based unit, and/or a mobile unit (e.g., a cellular phone, portable computer, etc.), implanted devices such as a pacemaker, cardioverter-defibrillator, loop recorder, and the like.

### Analyte Sensors

Analyte sensors herein can be of various types. In some embodiments, the physiological concentration of an analyte is sensed directly. In other embodiments, the physiological concentration of an analyte is sensed indirectly. By way of example, a metabolite of a particular analyte can be sensed instead of the particular analyte itself. In other embodiments, an analyte can be chemically converted into another form in order to make the process of detection easier. By way of example, an enzyme can be used to convert an analyte into another compound that is easier to detect. For example, the hydrolysis of creatinine into ammonia and N-methylhydantoin can be catalyzed by creatinine deiminase and the resulting ammonia can be detected by an analyte sensor.

In some embodiments, analyte sensors herein can include at least two functional elements: a receptor and a transducer. It will be appreciated that other elements can also be included. The receptor part of an analyte sensor can transform analyte information into a form of energy or signal that can be measured by the transducer. The transducer can transform and/or convey the energy or signal carrying the analyte information so as to provide a useful analytical signal.

Analyte sensors can include optical devices that utilize changes of optical phenomena or properties, which are the result of an interaction of the analyte with the receptor part of the sensor. Such optical properties can include: absorbance, caused by the absorptivity of the analyte itself or by a reaction with some suitable indicator; reflectance, using a bodily component, tissue, or fluid, or using an immobilized indicator; photoluminescence, based on the measurement of the intensity of light emitted by a chemical reaction in the receptor system; fluorescence, measured as the positive emission effect caused by irradiation or selective quenching of fluorescence; refractive index, measured as the result of a change in solution composition, in some cases including surface plasmon resonance effects; optothermal effects, based on a measurement of the thermal effect caused by light absorption; light scattering; or the like. In some embodiments, optical analyte sensors can include an optode.

Analyte sensors can also include electrochemical devices that transform the effect of the electrochemical interaction between an analyte and an electrode into a useful signal. Such sensors can include voltammetric sensors, including amperometric devices. Also included are sensors based on chemically inert electrodes, chemically active electrodes and modified electrodes. Also included are sensors with and without (galvanic sensors) a current source. Sensors can also include potentiometric sensors, in which the potential of the indicator electrode (ion-selective electrode, redox electrode, metal oxide electrode, or the like) is measured against a reference electrode. Sensors can include chemically sensitized field effect transistors (CHEMFET) in which the effect of the interaction between the analyte and the active coating is transformed into a change of the source-drain current. Sensors can include potentiometric solid electrolyte gas sensors.

Analyte sensors can also include electrical devices based on measurements, where no electrochemical processes take place, but the signal arises from the change of electrical properties caused by interaction with the analyte. Such sensors can include metal oxide semiconductor sensors based on reversible redox processes of analyte gas components, organic semiconductor sensors, based on the formation of charge transfer complexes, which modify the charge carrier density, electrolytic conductivity sensors, and electric permittivity sensors.

Analyte sensors can also include mass sensitive devices that transform the mass change at a specially modified surface into a change of a property of the support material. The mass change can be caused by accumulation of the analyte. Such sensors can include piezoelectric devices based on the measurement the frequency change of the quartz oscillator plate caused by adsorption of a mass of the analyte at the oscillator and surface acoustic wave devices that depend on the modification of the propagation velocity of a generated acoustical wave affected by the deposition of a definite mass of the analyte.

Analyte sensors can also include magnetic devices based on the change of paramagnetic properties of a gas being analyzed. Analyte sensors can also include thermometric devices based on the measurement of the heat effects of a specific chemical reaction or adsorption that involves the analyte.

In one example of the operation of an optical analyte sensor, analytes of interest from the in vivo environment can diffuse into an analyte sensing element causing a detectable change in the optical properties of the analyte sensing element. Light can be generated by an optical excitation device or emitter, such as an LED or similar device, and can pass through the optical window and into the analyte sensing element. Light can then either be preferentially reflected from or re-emitted by the analyte sensing element proportionally to the sensed analyte and pass back through the optical window before being received by a light detection device or receiver, such as a charge-coupled device (CCD), a photodiode, a junction field effect transistor (JFET) type optical sensor, of complementary metal-oxide semiconductor (CMOS) type optical sensor. Various aspects of exemplary analyte sensors are described in greater detail in U.S. Pat. No. 7,809,441. In another example of the operation of an optical analyte sensor, the optical properties of a tissue or fluid in the body can be directly analyzed. By way of example, light can be generated by an optical excitation device that can be delivered to a component, tissue, or fluid in the body and a light detection device can be used to sense an optical property of the light that has interfaced with the component, tissue, or fluid.

In accordance with the embodiments herein, some sensing element(s) can include one or more ion-selective sensors. Ion-selective sensors may either rely on surface phenomena or on concentration changes inside the bulk of a phase. Ion-selective sensors can include optical sensors, including both non-carrier optical sensors and carrier-based optical sensors, and ion-selective electrodes (ISEs). In some embodiments, the ion-selective sensor is fluorimetric, and can include a complexing moiety and a fluorescing moiety. Fluorimetric ion-selective sensors can exhibit differential fluorescent intensity based upon the complexing of an analyte to a complexing moiety. In some embodiments, the ion-selective sensor can be colorimetric, and can include a complexing moiety and a colorimetric moiety. Colorimetric ion-selective sensors can exhibit differential light absorbance based upon the complexing of an analyte to a complexing moiety.

In some embodiments, the ion-selective sensor comprises a non-carrier or carrier-based fluorescent or colorimetric ionophoric composition that comprises a complexing moiety for reversibly binding an ion to be analyzed, and a fluorescing or colorimetric moiety that changes its optical properties as the complexing agent binds or releases the ion. The complexing agents of the invention can optionally be appended with one or more organic substituents chosen to confer desired properties useful in formulating the ion sensing composition. By way of example, the substituents can be selected to stabilize the complexing agent with respect to leaching into the solution to be sensed, for example, by incorporating a hydrophobic or polymeric tail or by providing a means for covalent attachment of the complexing agent to a polymer support within the ion-selective sensor.

In some embodiments, the sensing element can include ion-selective sensors such as an ionophore or a fluorionophore. Suitable ionophores for use with the embodiments herein can include, but are not limited to, sodium specific ionophores, potassium specific ionophores, calcium specific ionophores, magnesium specific ionophores, and lithium specific ionophores. Suitable fluorionophores for use with the embodiments herein can include, but are not limited to, lithium specific fluoroionophores, sodium specific fluoroionophores, and potassium specific fluoroionophores.

Exemplary ion-selective sensors and methods for their use are disclosed in commonly assigned U.S. Pat. No. 7,809,441.

### Colorimetric and Photoluminescent Chemistries

Colorimetric response elements herein can be specific for a particular chemical analyte. Colorimetric response elements can include an element that changes color based on binding with or otherwise complexing with a specific chemical analyte. In some embodiments, a colorimetric response element can include a complexing moiety and a colorimetric moiety. Those moieties can be a part of a single chemical compound (as an example a non-carrier based system) or they can be separated on two or more different chemical compounds (as an example a carrier based system). The colorimetric moiety can exhibit differential light absorbance on binding of the complexing moiety to an analyte.

Photoluminescent response elements herein can be specific for a particular chemical analyte. Photoluminescent response elements herein can include an element that absorbs and emits light through a photoluminescent process, wherein the intensity and/or wavelength of the emission is impacted based on binding with or otherwise complexing with a specific chemical analyte. In some embodiments, a photoluminescent response element can include a complexing moiety and a fluorescing moiety. Those moieties can be a part of a single chemical compound (as an example a non-carrier based system) or they can be separated on two or more different chemical compounds (as an example a carrier based system). In some embodiments, the fluorescing moiety can exhibit different fluorescent intensity and/or emission wavelength based upon binding of the complexing moiety to an analyte.

Some chemistries may not require a separate compound to both complex an analyte of interest and produce an optical response. By way of example, in some embodiments, the response element can include a non-carrier optical moiety or material wherein selective complexation with the analyte of interest directly produces either a colorimetric or fluorescent response. As an example, a fluoroionophore can be used and is a compound including both a fluorescent moiety and an ion complexing moiety. As merely one example, (6,7-[2.2.2]-cryptando-3-[2"-(5"-carboethoxy)thiophenyl]coumarin, a potassium ion selective fluoroionophore, can be used (and in some cases covalently attached to polymeric matrix or membrane) to produce a fluorescence-based K⁺ non-carrier response element.

An exemplary class of fluoroionophores are the coumarocryptands. Coumarocryptands can include lithium specific fluoroionophores, sodium specific fluoroionophores, and potassium specific fluoroionophores. For example, lithium specific fluoroionophores can include (6,7-[2.1.1]-cryptando-3-[2"-(5"-carboethoxy)furyl]coumarin. Sodium specific fluoroionophores can include (6,7-[2.2.1]-cryptando-3-[2"-(5"-carboethoxy)furyl]coumarin. Potassium specific fluoroionophores can include (6,7-[2.2.2]-cryptando-3-[2"-(5"-carboethoxy)furyl]coumarin and (6,7-[2.2.2]-cryptando-3-[2"-(5"-carboethoxy)thiophenyl]coumarin.

Suitable fluoroionophores include the coumarocryptands taught in U.S. Pat. No. 5,958,782. Such fluorescent ionophoric compounds can be excited with GaN blue light emitting diodes (LEDs) emitting light at or about 400 nm. These fluorescent ionophoric compounds have ion concentration dependent emission that can be detected in the wavelength range of about 450 nm to about 470 nm.

Some chemistries can rely upon a separate complexing entity (e.g., a separate chemical compound). As an example, carrier based response elements can include a compound, in some cases referred to as an ionophore, that complexes with and serves to carry the analyte of interest. In some embodiments, carrier based response elements include a lipophilic ionophore, and a lipophilic fluorescent or colorimetric indicator dye, called a chromoionophore. In some cases the chromoionophore and the ionophore can be dispersed in, and/or covalently attached to, a hydrophobic organic polymeric matrix. The ionophore can be capable of reversibly binding ions of interest. The chromoionophore can be a proton selective dye. In operation, analytes of interest are reversibly sequestered by the ionophores within the organic polymer matrix. To maintain charge neutrality within the polymer matrix, protons are then released from the chromoionophore, giving rise to a color or fluorescence change. As just one specific example, a carrier based response element can include potassium ionophore III, chromoionophore I, and potassium tetrakis(4-chlorophenyl)borate dispersed in a polymer matrix made from polyvinylchloride and bis(2-ethylhexyl)sebacate surfactant to produce a colorimetric K⁺ sensing element.

Both non-carrier based response elements and carrier-based response elements can include complexing moieties. Suitable complexing moieties can include cryptands, crown ethers, bis-crown ethers, calixarenes, noncyclic amides, and hemispherand moieties as well as ion selective antibiotics such as monensin, valinomycin and nigericin derivatives.

Those of skill in the art can recognize which cryptand and crown ether moieties are useful in complexing particular cations, although reference can be made to, for example, Lehn and Sauvage, "[2]-Cryptates: Stability and Selectivity of Alkali and Alkaline-Earth Macrocyclic Complexes," J. Am. Chem. Soc, 97, 6700-07 (1975), for further information on this topic. Those skilled in the art can recognize which bis-crown ether, calixarene, noncyclic amides, hemispherand, and antibiotic moieties are useful in complexing particular cations, although reference can be made to, for example, Buhlmann et al., "Carrier-Based Ion-Selective Electrodes and Bulk Optodes. 2. Ionophores for Potentiometric and Optical Sensors," Chem. Rev. 98, 1593-1687 (1998), for further information on this topic.

By way of example cryptands can include a structure referred to as a cryptand cage. For cryptand cages, the size of the cage is defined by the oxygen and nitrogen atoms and the size makes cryptand cages quite selective for cations with a similar diameter. For example a [2.2.2] cryptand cage is quite selective for cations such as K⁺, Pb⁺², Sr⁺², and Ba⁺². A [2.2.1] cryptand cage is quite selective for cations such as Na⁺ and Ca⁺². Finally, a [2.1.1] cryptand cage is quite selective for cations such as Li⁺ and Mg⁺². The size selectivity of cryptand cages can aid in the sensitivity of chemical sensing. When these cryptand cages are incorporated into physiologic sensing systems heavier metals such as Pb⁺² and Ba⁺² are unlikely to be present in concentrations which interfere with the analysis of ions of broader physiological interest such as Na⁺ and K⁺.

In some embodiments, sensing elements can operate using multiple non-overlapping fluorescence modes. For example, two or more fluorescent indicators can be used with one triggered by a first wavelength "A" of light and one triggered by a different wavelength "B" of light. Many combinations of colorimetric and photoluminescent sensing elements are contemplated herein.

Further aspects of colorimetric and photoluminescent sensors and components thereof are described in U.S. Pat. Nos. 7,809,441 and 8,126,554.

### Adjustment for Intramodal Interference

As described above, the circuitry can be configured to calculate a concentration of two or more analytes using signals received from the optical detection assembly. In particular, the system can include a multimodal chemical sensor (such as one operating both colorimetrically and photoluminescently, or one operating photoluminescently (such as fluorescently) but at two different wavelengths) so that multiple analytes can be detected with a single sensing element (thought the device may have more than one sensing element). However, it is known that in some circumstances colorimetric sensing components can absorb light at frequencies associated with the response of a photoluminescent sensing element. As such, the colorimetric sensing components can interfere with photoluminescent sensing in some cases. However, by characterizing the interference across frequencies and amplitudes it is possible to correct the photoluminescent signal for this effect or account for it.

In some embodiments, the circuitry can process a signal from the photoluminescent response element using a signal from the colorimetric response element as an input. In some embodiments, the control circuit is configured to calculate a concentration of the first chemical analyte based on a signal from the photoluminescent response element, wherein the control circuit is configured to increase or decrease the calculated concentration based on a signal from the colorimetric response element.

The amount of interference provided by the colorimetric components can change based on the colorimetric state of the colorimetric components, which itself is influenced by the concentration of analyte for which the colorimetric sensing components are specific. Therefore, in some embodiments, a map or correlation table can be generated that relates colorimetric analyte concentrations to absorption of electromagnetic waves at photoluminescent response frequencies to determine an expected absorption of electromagnetic waves at photoluminescent response frequencies for calculated concentrations of the colorimetric analyte.

In some embodiments, the implantable medical device or a portion thereof such as a control circuit can be configured to receive a colorimetric signal from the colorimetric response element, calculate a concentration of the analyte for which the colorimetric response element is specific using the received colorimetric signal and use a map that relates colorimetric analyte concentrations to absorption of electromagnetic waves at photoluminescent response frequencies to determine an expected absorption of electromagnetic waves at photoluminescent response frequencies for the calculated concentration of the colorimetric analyte.

In some embodiments, the implantable medical device or a portion thereof such as a control circuit can be further configured to receive a photoluminescent signal from the photoluminescent response element, add or otherwise account for the expected absorption of electromagnetic waves at photoluminescent response frequencies to the received photoluminescent signal to generate a corrected photoluminescent signal and calculate a concentration of the analyte for with the photoluminescent response element is specific using the corrected photoluminescent signal.

In some embodiments, the implantable medical device or a portion thereof such as a control circuit can be further configured to receive a colorimetric signal from the colorimetric response element and use a correlation table that relates colorimetric signals to absorption of electromagnetic waves at photoluminescent response frequencies to determine an expected absorption of electromagnetic waves for the received colorimetric signal.

### Optical Excitation and Detection Assemblies

In some embodiments, the optical excitation assembly can include solid state light sources such as GaAs, GaAlAs, GaAlAsP, GaAlP, GaAsP, GaP, GaN, InGaAlP, InGaN, ZnSe, or SiC light emitting diodes or laser diodes that excite the sensing element(s) at or near the wavelength of maximum absorption for a time sufficient to emit a return signal. However, it will be understood that in some embodiments the wavelength of maximum absorption/reflection varies as a function of concentration in the colorimetric sensor.

In some embodiments, the optical excitation assemblies can include other light emitting components including incandescent components. In some embodiments, the optical excitation assemblies can include a waveguide. The optical excitation assemblies can also include one or more optical filters such as bandpass filters, high pass filters, low pass filters, or antireflection elements, and/or focusing optics.

In some embodiments, the optical excitation assemblies can include a plurality of LEDs with bandpass filters, each of the LED-filter combinations emitting at a different center frequency. According to various embodiments, the LEDs can operate at different center-frequencies, sequentially turning on and off during a measurement, illuminating the sensing element(s). As multiple different center-frequency measurements are made sequentially, a single unfiltered detector can be used in some embodiments. However, in some embodiments, a polychromatic source can be used with multiple detectors that are each bandpass filtered to a particular center frequency.

The sensing element(s) can include one or more types of indicator beads having embedded therein various types of ion-selective sensors. Physiological analytes of interest can diffuse into and out of the sensing element(s) and bind with an ion-selective sensor to result in a fluorimetric or colorimetric response. Reference analytes can similarly diffuse into and out of the sensing element(s) and serve as a control sample. Exemplary ion-selective sensors are described more fully below.

The optical detection assembly can be configured to receive light from the sensing element(s). In an embodiment, the optical detection assembly can include a component to receive light. By way of example, in some embodiments, the optical detection assembly can include a charge-coupled device (CCD). In other embodiments, the optical detection assembly can include a photodiode, a junction field effect transistor (JFET) type optical sensor, or a complementary metal-oxide semiconductor (CMOS) type optical sensor. In some embodiments, the optical detection assembly can include an array of optical sensing components. In some embodiments, the optical detection assembly can include a waveguide. In some embodiments, the optical detection assembly can also include one or more bandpass filters and/or focusing optics. In some embodiments, the optical detection assembly can include one or more photodiode detectors, each with an optical bandpass filter tuned to a specific wavelength range.

The optical excitation and detection assemblies embodied herein, can be integrated using bifurcated fiber-optics that direct excitation light from a light source to one or more sensing element(s), or simultaneously to sensing element(s) and a reference channel. Return fibers can direct emission signals from the sensing element(s) and the reference channels to one or more optical detection assemblies for analysis by a processor, such as a microprocessor. In some embodiments, the optical excitation and optical detection assemblies are integrated using a beam-splitter assembly and focusing optical lenses that direct excitation light from a light source to the sensing element and direct emitted or reflected light from the sensing element to an optical detector for analysis by a processor.

### Ion-Permeable Polymeric Matrix Materials

As referenced above, the analyte window and/or outer barrier layer of each sensing element can be formed of an ion-permeable polymeric matrix material in some embodiments. Suitable polymers for use as the ion-permeable polymeric matrix material can include, but are not limited to polymers forming a hydrogel. Hydrogels herein can include homopolymeric hydrogels, copolymeric hydrogels, and multipolymer interpenetrating polymeric hydrogels. Hydrogels herein can specifically include nonionic hydrogels. In some embodiments, hydrogels herein can be prepared from polymerization of various monomers or macromers including one or more of 2-hydroxyethyl methacrylate (HEMA), 2-hydroxypropyl methacrylate (HPMA), acrylamide, acrylic acid, N-isopropylacrylamide (NIPAm), methoxyl polyethylene glycol monoacrylate (PEGMA), and the like. In some embodiments, polymers can include, but are not limited to polyhydroxyethyl methacrylate (polyHEMA), cellulose, polyvinyl alcohol, dextran, polyacrylamides, polyhydroxyalkyl acrylates, polyvinyl pyrrolidones, and mixtures and copolymers thereof. In some embodiments, suitable polymers for use with the ion-permeable polymeric matrix described herein include those that are transparent.

### Physiological Analvtes

Examples of physiological analytes that can be measured in accordance with chemical sensors of embodiments herein can include, but are not limited to, electrolytes, hormones, proteins, sugars, metabolites, and the like.

Chemical sensors herein can be directed at a specific analyte or a plurality of different analytes. In an embodiment, the analyte sensed is one or more analytes relevant to cardiac health. In an embodiment, the analyte sensed is one or more analytes indicative of renal health. The analyte sensed can be an ion or a non-ion. The analyte sensed can be a cation or an anion. Specific examples of analytes that can be sensed include acetic acid (acetate), aconitic acid (aconitate), ammonium, blood urea nitrogen (BUN), B-type natriuretic peptide (BNP), bromate, calcium, carbon dioxide, cardiac specific troponin, chloride, choline, citric acid (citrate), cortisol, copper, creatinine, creatinine kinase, fluoride, formic acid (formate), glucose, hydronium ion, isocitrate, lactic acid (lactate), lithium, magnesium, maleic acid (maleate), malonic acid (malonate), myoglobin, nitrate, nitric-oxide, oxalic acid (oxalate), oxygen, phosphate, phthalate, potassium, pyruvic acid (pyruvate), selenite, sodium, sulfate, urea, uric acid, and zinc. Inorganic cations sensed by this method include but not limited to hydronium ion, lithium ion, sodium ion, potassium ion, magnesium ion, calcium ion, silver ion, zinc ion, mercury ion, lead ion and ammonium ion. Inorganic anions sensed by this method include but not limited to carbonate anion, nitrate anion, sulfite anion, chloride anion and iodide anion. Organic cations sensed by this method include but are not limited to norephedrine, ephedrine, amphetamine, procaine, prilocaine, lidocaine, bupivacaine, lignocaine, creatinine and protamine. Organic anions sensed by this method include but not limited to salicylate, phthalate, maleate, and heparin. Neutral analytes sensed by this method include but not limited to ammonia, ethanol, and organic amines. In an embodiment, ions that can be sensed include potassium, sodium, chloride, calcium, and hydronium (pH). In a particular embodiment, concentrations of both sodium and potassium are measured. In another embodiment, concentrations of both magnesium and potassium are measured.

In some embodiments, the analytes can specifically include one or more of sodium ion, magnesium ion, chloride ion, calcium ion, carbonate ion, phosphate ion, sulfate ion, insulin, aldosterone, troponin, glucose, creatinine, and BNP.

In some embodiments, the analytes can specifically include one or more of partial pressure of oxygen (PaO₂), partial pressure of carbon dioxide (PaCO₂) and oxygen saturation (O₂Sat).

All publications and patent applications in this specification are indicative of the level of ordinary skill in the art to which this invention pertains.

## Claims

1. An implantable medical device (100) comprising:
an optical excitation assembly (312, 313);
an optical detection assembly (314); and a multimode sensing element (402) comprising:
an outer barrier layer (404) forming a top (308), a bottom (406), and opposed sides (408, 410) of the sensing element, the outer barrier layer (404) defining an interior volume;
a colorimetric response element specific for a first chemical analyte;
a photoluminescent response element specific for a second chemical analyte;
the interior volume comprising a first chamber (602) and a second chamber (604) separate from the first chamber, wherein the colorimetric response element (422) is disposed within the first chamber (602) and the photoluminescent response element (424) is disposed within the second chamber (604),
wherein the first chamber (602) and the second chamber (604) are arranged side-by-side, the optical excitation and optical detection assemblies (312, 313, 314) being located on opposite lateral sides of the sensing element (402).

2. The implantable medical device (100) of claim 1, wherein the photoluminescent response element is fluorescent.

3. The implantable medical device (100) of any of claims 1-2, wherein the colorimetric response element is specific for potassium ion and the photoluminescent response element is specific for glucose.

4. The implantable medical device (100) of any of claims 1-3,
wherein the colorimetric response element and the photoluminescent response element are both disposed on or within the polymeric beads.

5. The implantable medical device (100) of any of claims 1-4, wherein the optical excitation assembly (312, 313) comprising a first electromagnetic wave emitter emitting light at a first primary frequency and a second electromagnetic wave emitter emitting light at a second primary frequency.

6. The implantable medical device (100) of any of claims 1-5, further comprising a control circuit (908) configured to receive a signal from the optical detection assembly (314), wherein the control circuit is configured to process a signal from the photoluminescent response element using a signal from the colorimetric response element as an input.

7. The implantable medical device (100) of any of claims 1-6, further comprising a control circuit configured to receive a signal from the optical detection assembly (314), wherein the control circuit is configured to calculate a concentration of the first chemical analyte based on a signal from the photoluminescent response element, wherein the control circuit is configured to increase or decrease the calculated concentration based on a signal from the colorimetric response element.

8. The implantable medical device (100) of any of claims 1-7, the colorimetric response element specific for a first chemical analyte comprising a complexing moiety and a colorimetric moiety, the colorimetric moiety exhibiting differential light absorbance on binding of the complexing moiety to an analyte.

9. The implantable medical device (100) of any of claims 1-8, the photoluminescent response element specific for a first chemical analyte comprising a complexing moiety and a fluorescing moiety, the fluorescing moiety exhibiting different fluorescent intensity based upon binding of the complexing moiety to an analyte.

10. The implantable medical device (100) of any of claims 1-9, further comprising a control circuit configured to receive a signal from the optical detection assembly (314), wherein the control circuit is configured to:
receive a colorimetric signal from the colorimetric response element;
calculate a concentration of the analyte for which the colorimetric response element is specific using the received colorimetric signal; and
use a map that relates colorimetric analyte concentrations to absorption of electromagnetic waves at photoluminescent response frequencies to determine an expected absorption of electromagnetic waves at photoluminescent response frequencies for the calculated concentration of the colorimetric analyte.

11. The implantable medical device (100) of any of claims 1-10, further comprising a control circuit configured to receive a signal from the optical detection assembly (314), wherein the control circuit is further configured to:
receive a photoluminescent signal from the photoluminescent response element;
add the expected absorption of electromagnetic waves at photoluminescent response frequencies to the received photoluminescent signal to generate a corrected photoluminescent signal; and
calculate a concentration of the analyte for with the photoluminescent response element is specific using the corrected photoluminescent signal.

12. The implantable medical device (100) of any of claims 1-11, further comprising a control circuit configured to receive a signal from the optical detection assembly (314), wherein the control circuit is configured to:
receive a colorimetric signal from the colorimetric response element; and
use a correlation table that relates colorimetric signals to absorption of electromagnetic waves at photoluminescent response frequencies to determine an expected absorption of electromagnetic waves for the received colorimetric signal.

## Patentansprüche

1. Implantierbare medizinische Vorrichtung (100), umfassend:
eine optische Anregungsanordnung (312, 313);
eine optische Detektionsanordnung (314); und
ein multimodales Abtastelement (402), umfassend:
eine äußere Sperrschicht (404), ausbildend eine Oberseite (308), eine Unterseite (406) und gegenüberliegende Seiten (408, 410) des Abtastelements, wobei die äußere Sperrschicht (404) ein Innenvolumen definiert;
ein colorimetrisches Reaktionselement, spezifisch für einen ersten chemischen Analyten;
ein photolumineszentes Reaktionselement, spezifisch für einen zweiten chemischen Analyten;
wobei das Innenvolumen eine erste Kammer (602) und eine von der ersten Kammer getrennte zweite Kammer (604) umfasst, wobei das colorimetrische Reaktionselement (422) innerhalb der ersten Kammer (602) angeordnet ist und das photolumineszente Reaktionselement (424) innerhalb der zweiten Kammer (604) angeordnet ist,
wobei die erste Kammer (602) und die zweite Kammer (604) nebeneinander angeordnet sind, wobei die optische Anregungs- und die optische Detektionsanordnung (312, 313, 314) auf gegenüberliegenden lateralen Seiten des Abtastelements (402) angeordnet sind.

2. Implantierbare medizinische Vorrichtung (100) nach Anspruch 1, wobei das photolumineszente Reaktionselement fluoreszent ist.

3. Implantierbare medizinische Vorrichtung (100) nach einem der Ansprüche 1-2, wobei das colorimetrische Reaktionselement spezifisch für ein Kaliumion ist und das photolumineszente Reaktionselement spezifisch für Glukose ist.

4. Implantierbare medizinische Vorrichtung (100) nach einem der Ansprüche 1-3,
wobei das colorimetrische Reaktionselement und das photolumineszente Reaktionselement beide an den oder innerhalb der Polymerkugeln angeordnet sind.

5. Implantierbare medizinische Vorrichtung (100) nach einem der Ansprüche 1-4, wobei die optische Anregungsanordnung (312, 313) einen ersten Emitter elektromagnetischer Wellen, der Licht in einer ersten Primärfrequenz emittiert, und einen zweiten Emitter elektromagnetischer Wellen, der Licht in einer zweiten Primärfrequenz emittiert, umfasst.

6. Implantierbare medizinische Vorrichtung (100) nach einem der Ansprüche 1-5, ferner umfassend eine Steuerschaltung (908), dazu ausgelegt, ein Signal von der optischen Detektionsanordnung (314) zu empfangen, wobei die Steuerschaltung dazu ausgelegt ist, ein Signal von dem photolumineszenten Reaktionselement unter Verwendung eines Signals von dem colorimetrischen Reaktionselement als einen Eingang zu empfangen.

7. Implantierbare medizinische Vorrichtung (100) nach einem der Ansprüche 1-6, ferner umfassend eine Steuerschaltung, dazu ausgelegt, ein Signal von der optischen Detektionsanordnung (314) zu empfangen, wobei die Steuerschaltung dazu ausgelegt ist, eine Konzentration des ersten chemischen Analyten auf Grundlage eines Signals von dem photolumineszenten Reaktionselement zu berechnen, wobei die Steuerschaltung dazu ausgelegt ist, die berechnete Konzentration auf Grundlage eines Signals von dem colorimetrischen Reaktionselement zu erhöhen oder zu verringern.

8. Implantierbare medizinische Vorrichtung (100) nach einem der Ansprüche 1-7, wobei das für einen ersten chemischen Analyten spezifische colorimetrische Reaktionselement eine komplexbildende Komponente und eine colorimetrische Komponente umfasst, wobei die colorimetrische Komponente bei Bindung der komplexbildenden Komponente an einen Analyten eine unterschiedliche Lichtextinktion aufweist.

9. Implantierbare medizinische Vorrichtung (100) nach einem der Ansprüche 1-8, wobei das für einen ersten chemischen Analyten spezifische photolumineszente Reaktionselement eine komplexbildende Komponente und eine fluoreszierende Komponente umfasst, wobei die fluoreszierende Komponente auf Grundlage der Bindung der komplexbildenden Komponente an einen Analyten eine unterschiedliche Fluoreszenzintensität aufweist.

10. Implantierbare medizinische Vorrichtung (100) nach einem der Ansprüche 1-9, ferner umfassend eine Steuerschaltung, dazu ausgelegt, ein Signal von der optischen Detektionsanordnung (314) zu empfangen, wobei die Steuerschaltung dazu ausgelegt ist:
ein colorimetrisches Signal von dem colorimetrischen Reaktionselement zu empfangen;
unter Verwendung des empfangenen colorimetrischen Signals eine Konzentration des Analyten zu berechnen, für die das colorimetrische Reaktionselement spezifisch ist; und
eine Abbildung zu verwenden, die colorimetrische Analytkonzentrationen in ein Verhältnis zur Absorption elektromagnetischer Wellen bei photolumineszenten Reaktionsfrequenzen setzt, um eine erwartete Absorption elektromagnetischer Wellen bei photolumineszenten Reaktionsfrequenzen für die berechnete Konzentration des colorimetrischen Analyten zu bestimmen.

11. Implantierbare medizinische Vorrichtung (100) nach einem der Ansprüche 1-10, ferner umfassend eine Steuerschaltung, dazu ausgelegt, ein Signal von der optischen Detektionsanordnung (314) zu empfangen, wobei die Steuerschaltung ferner dazu ausgelegt ist:
ein photolumineszentes Signal von dem photolumineszenten Reaktionselement zu empfangen;
die erwartete Absorption elektromagnetischer Wellen bei photolumineszenten Reaktionsfrequenzen zu dem empfangenen photolumineszenten Signal zu addieren, um ein korrigiertes photolumineszentes Signal zu generieren; und
unter Verwendung des korrigierten photolumineszenten Signals eine Konzentration des Analyten zu berechnen, für die das photolumineszente Reaktionselement spezifisch ist.

12. Implantierbare medizinische Vorrichtung (100) nach einem der Ansprüche 1-11, ferner umfassend eine Steuerschaltung, dazu ausgelegt, ein Signal von der optischen Detektionsanordnung (314) zu empfangen, wobei die Steuerschaltung dazu ausgelegt ist:
ein colorimetrisches Signal von dem colorimetrischen Reaktionselement zu empfangen; und
eine Korrelationstabelle zu verwenden, die colorimetrische Signale in ein Verhältnis zur Absorption elektromagnetischer Wellen bei photolumineszenten Reaktionsfrequenzen setzt, um für das empfangene colorimetrische Signal eine erwartete Absorption elektromagnetischer Wellen zu bestimmen.

## Revendications

1. Dispositif médical implantable (100) comprenant :
un assemblage d'excitation optique (312, 313) ;
un assemblage de détection optique (314) ; et
un élément de détection à multiples modes (402) qui comprend :
une couche de barrière externe (404) qui forme un sommet (308), un fond (406) et des côtés opposés (408, 410) de l'élément de détection, la couche de barrière externe (404) définissant un volume intérieur ;
un élément de réponse colorimétrique qui est spécifique pour un premier analyte chimique ; et
un élément de réponse photoluminescent qui est spécifique pour un second analyte chimique ;
le volume intérieur comprenant une première chambre (602) et une seconde chambre (604) qui est séparée de la première chambre, dans lequel l'élément de réponse colorimétrique (422) est disposé à l'intérieur de la première chambre (602) et l'élément de réponse photoluminescent (424) est disposé à l'intérieur de la seconde chambre (604),
dans lequel la première chambre (602) et la seconde chambre (604) sont agencées côte à côte, les assemblages d'excitation optique et de détection optique (312, 313, 314) étant localisés sur des côtés latéraux opposés de l'élément de détection (402).

2. Dispositif médical implantable (100) selon la revendication 1, dans lequel l'élément de réponse photoluminescent est fluorescent.

3. Dispositif médical implantable (100) selon l'une quelconque des revendications 1 et 2, dans lequel l'élément de réponse colorimétrique est spécifique pour l'ion potassium et l'élément de réponse photoluminescent est spécifique pour le glucose.

4. Dispositif médical implantable (100) selon l'une quelconque des revendications 1 à 3,
dans lequel l'élément de réponse colorimétrique et l'élément de réponse photoluminescent sont tous deux disposés sur ou à l'intérieur des billes en polymère.

5. Dispositif médical implantable (100) selon l'une quelconque des revendications 1 à 4, dans lequel l'assemblage d'excitation optique (312, 313) comprend un premier émetteur d'ondes électromagnétiques qui émet de la lumière à une première fréquence primaire et un second émetteur d'ondes électromagnétiques qui émet de la lumière à une seconde fréquence primaire.

6. Dispositif médical implantable (100) selon l'une quelconque des revendications 1 à 5, comprenant en outre un circuit de commande (908) qui est configuré pour recevoir un signal en provenance de l'assemblage de détection optique (314), dans lequel le circuit de commande est configuré pour traiter un signal en provenance de l'élément de réponse photoluminescent en utilisant un signal en provenance de l'élément de réponse colorimétrique en tant qu'entrée.

7. Dispositif médical implantable (100) selon l'une quelconque des revendications 1 à 6, comprenant en outre un circuit de commande qui est configuré pour recevoir un signal en provenance de l'assemblage de détection optique (314), dans lequel le circuit de commande est configuré pour calculer une concentration du premier analyte chimique sur la base d'un signal en provenance de l'élément de réponse photoluminescent, et dans lequel le circuit de commande est configuré pour augmenter ou diminuer la concentration calculée sur la base d'un signal en provenance de l'élément de réponse colorimétrique.

8. Dispositif médical implantable (100) selon l'une quelconque des revendications 1 à 7, l'élément de réponse colorimétrique spécifique pour un premier analyte chimique comprenant une moitié de complexion et une moitié colorimétrique, la moitié colorimétrique présentant une absorbance de lumière différentielle suite à la liaison de la moitié de complexion sur un analyte.

9. Dispositif médical implantable (100) selon l'une quelconque des revendications 1 à 8, l'élément de réponse photoluminescent spécifique pour un premier analyte chimique comprenant une moitié de complexion et une moitié fluorescente, la moitié fluorescente présentant une intensité fluorescente différente sur la base de la liaison de la moitié de complexion sur un analyte.

10. Dispositif médical implantable (100) selon l'une quelconque des revendications 1 à 9, comprenant en outre un circuit de commande qui est configuré pour recevoir un signal en provenance de l'assemblage de détection optique (314), dans lequel le circuit de commande est configuré pour :
recevoir un signal colorimétrique en provenance de l'élément de réponse colorimétrique ;
calculer une concentration de l'analyte pour lequel l'élément de réponse colorimétrique est spécifique en utilisant le signal colorimétrique reçu ; et
utiliser une carte qui rapporte les concentrations d'analyte colorimétrique à l'absorption d'ondes électromagnétiques pour des fréquences de réponse photoluminescente pour déterminer une absorption attendue d'ondes électromagnétiques pour des fréquences de réponse photoluminescente pour la concentration calculée de l'analyte colorimétrique.

11. Dispositif médical implantable (100) selon l'une quelconque des revendications 1 à 10, comprenant en outre un circuit de commande qui est configuré pour recevoir un signal en provenance de l'assemblage de détection optique (314), dans lequel le circuit de commande est en outre configuré pour :
recevoir un signal photoluminescent en provenance de l'élément de réponse photoluminescent ;
ajouter l'absorption attendue d'ondes électromagnétiques pour des fréquences de réponse photoluminescente au signal photoluminescent reçu pour générer un signal photoluminescent corrigé ; et
calculer une concentration de l'analyte pour lequel l'élément de réponse photoluminescent est spécifique en utilisant le signal photoluminescent corrigé.

12. Dispositif médical implantable (100) selon l'une quelconque des revendications 1 à 11, comprenant en outre un circuit de commande qui est configuré pour recevoir un signal en provenance de l'assemblage de détection optique (314), dans lequel le circuit de commande est configuré pour :
recevoir un signal colorimétrique en provenance de l'élément de réponse colorimétrique ; et
utiliser une table de corrélation qui rapporte les signaux colorimétriques à l'absorption d'ondes électromagnétiques pour des fréquences de réponse photoluminescente pour déterminer une absorption attendue d'ondes électromagnétiques pour le signal colorimétrique reçu.
